# EUROPEAN PATENT SPECIFICATION

(11) **EP 4 188 477 B1**
(45) Date of publication and mention of the grant of the patent: **28.10.2026**
(21) Application number: 21758246.9
(22) Date of filing: 29.07.2021
(51) Int. Cl.: A61M 1/28, A61M 1/14

(54) **PERITONEAL DIALYSIS CYCLER USING MICROPUMP**
PERITONEALDIALYSE-CYCLER MIT MIKROPUMPE
CYCLEUR DE DIALYSE PÉRITONÉALE À L'AIDE DE MICROPOMPE

(30) Priority: 03.08.2020 IN 202041033187
(43) Date of publication of application: 07.06.2023
(73) Proprietor: Vantive US Healthcare LLC, Deerfield, IL 60015 (US); Vantive Health GmbH, 8152 Glattpark (Opfikon) (CH)
(72) Inventor: THIRUMATTATHIL ASHOKAN, Anoop, Bangalore Karnataka 560 048 (IN); PITCHAIMANI, Karthik, Bangalore Karnataka 560 048 (IN); KAMATH, Sadashiva, Bangalore Karnataka 560 048 (IN); MURMU, Sankar, Bangalore Karnataka 560 048 (IN); NISHANTH IRUDHAYARAJ, Emmanuel Thomas, Bangalore Karnataka 560 048 (IN); MALAGOLI, Marcello, 41036 Medolla (IT)
(74) Representative: Potter Clarkson
(86) International application number: PCT/US2021/043614
(87) International publication number: WO 2022/031504

(56) References cited:
- EP-A1- 0 772 470
- EP-B1- 0 643 592
- AU-A1- 2013 222 017
- US-A1- 2006 189 923
- US-A1- 2011 160 649
- US-A1- 2017 340 794
- US-A1- 2019 224 400
- US-A1- 2019 321 535

## Description

### BACKGROUND

The present disclosure relates generally to medical fluid treatments and in particular to dialysis fluid treatments.

Due to various causes, a person's renal system can fail. Renal failure produces several physiological derangements. It is no longer possible to balance water and minerals or to excrete daily metabolic load. Toxic end products of metabolism, such as, urea, creatinine, uric acid and others, may accumulate in a patient's blood and tissue.

Reduced kidney function and, above all, kidney failure is treated with dialysis. Dialysis removes waste, toxins and excess water from the body that normal functioning kidneys would otherwise remove. Dialysis treatment for replacement of kidney functions is critical to many people because the treatment is lifesaving.

One type of kidney failure therapy is Hemodialysis ("HD"), which in general uses diffusion to remove waste products from a patient's blood. A diffusive gradient occurs across the semi-permeable dialyzer between the blood and an electrolyte solution called dialysate or dialysis fluid to cause diffusion.

Hemofiltration ("HF") is an alternative renal replacement therapy that relies on a convective transport of toxins from the patient's blood. HF is accomplished by adding substitution or replacement fluid to the extracorporeal circuit during treatment. The substitution fluid and the fluid accumulated by the patient in between treatments is ultrafiltered over the course of the HF treatment, providing a convective transport mechanism that is particularly beneficial in removing middle and large molecules.

Hemodiafiltration ("HDF") is a treatment modality that combines convective and diffusive clearances. HDF uses dialysis fluid flowing through a dialyzer, similar to standard hemodialysis, to provide diffusive clearance. In addition, substitution solution is provided directly to the extracorporeal circuit, providing convective clearance.

Most HD, HF, and HDF treatments occur in centers. A trend towards home hemodialysis ("HHD") exists today in part because HHD can be performed daily, offering therapeutic benefits over in-center hemodialysis treatments, which occur typically bi- or triweekly. Studies have shown that more frequent treatments remove more toxins and waste products and render less interdialytic fluid overload than a patient receiving less frequent but perhaps longer treatments. A patient receiving more frequent treatments does not experience as much of a down cycle (swings in fluids and toxins) as does an in-center patient, who has built-up two or three days' worth of toxins prior to a treatment. In certain areas, the closest dialysis center can be many miles from the patient's home, causing door-to-door treatment time to consume a large portion of the day. Treatments in centers close to the patient's home may also consume a large portion of the patient's day. HHD can take place overnight or during the day while the patient relaxes, works or is otherwise productive.

Another type of kidney failure therapy is peritoneal dialysis ("PD"), which infuses a dialysis solution, also called dialysis fluid, into a patient's peritoneal chamber via a catheter. The dialysis fluid is in contact with the peritoneal membrane in the patient's peritoneal chamber. Waste, toxins and excess water pass from the patient's bloodstream, through the capillaries in the peritoneal membrane, and into the dialysis fluid due to diffusion and osmosis, i.e., an osmotic gradient occurs across the membrane. An osmotic agent in the PD dialysis fluid provides the osmotic gradient. Used or spent dialysis fluid is drained from the patient, removing waste, toxins and excess water from the patient. This cycle is repeated, e.g., multiple times.

There are various types of peritoneal dialysis therapies, including continuous ambulatory peritoneal dialysis ("CAPD"), automated peritoneal dialysis ("APD"), tidal flow dialysis and continuous flow peritoneal dialysis ("CFPD"). CAPD is a manual dialysis treatment. Here, the patient manually connects an implanted catheter to a drain to allow used or spent dialysis fluid to drain from the peritoneal chamber. The patient then switches fluid communication so that the patient catheter communicates with a bag of fresh dialysis fluid to infuse the fresh dialysis fluid through the catheter and into the patient. The patient disconnects the catheter from the fresh dialysis fluid bag and allows the dialysis fluid to dwell within the peritoneal chamber, wherein the transfer of waste, toxins and excess water takes place. After a dwell period, the patient repeats the manual dialysis procedure, for example, four times per day. Manual peritoneal dialysis requires a significant amount of time and effort from the patient, leaving ample room for improvement.

Automated peritoneal dialysis ("APD") is similar to CAPD in that the dialysis treatment includes drain, fill and dwell cycles. APD machines, however, perform the cycles automatically, typically while the patient sleeps. APD machines free patients from having to manually perform the treatment cycles and from having to transport supplies during the day. APD machines connect fluidly to an implanted catheter, to a source or bag of fresh dialysis fluid and to a fluid drain. APD machines pump fresh dialysis fluid from a dialysis fluid source, through the catheter and into the patient's peritoneal chamber. APD machines also allow for the dialysis fluid to dwell within the chamber and for the transfer of waste, toxins and excess water to take place. The source may include multiple liters of dialysis fluid including several solution bags.

APD machines pump used or spent dialysate from the patient's peritoneal cavity, though the catheter, and to the drain. As with the manual process, several drain, fill and dwell cycles occur during dialysis. A "last fill" may occur at the end of the APD treatment. The last fill fluid may remain in the peritoneal chamber of the patient until the start of the next treatment, or may be manually emptied at some point during the day.

Known APD systems include a machine or cycler that accepts and actuates a pumping cassette having a hard part and a soft part that is deformable for performing pumping and valving operations. Sealing the fluid disposable cassette with a pneumatic path via a gasket to provide actuation has proven to be a potential field issue, which can delay treatment start time and affect use experience. Pneumatic cassette systems also produce acoustic noise, which may be a source of customer dissatisfaction.
US2006/0189923 describes a system and a method of use of said system for performing fluid administration on a patient, the system comprising: a liquid pump (1), a liquid distribution system (2) connected to said pump (1) in such a way that liquid can flow from the liquid distribution system (2) to the pump (1) and vice versa, liquid supply means (3) for supplying liquid to a patient (4) via said liquid distribution system (2) and said pump (1), a patient conduit (5) adapted for connecting said liquid distribution system (2) to a patient (4), the system being characterized by the fact that said liquid pump (1) is unidirectional and that said liquid distribution system (2) comprises switching means designed to alternatively connect the pump enter line (56) with the supply means (3) or with the patient conduit (5).
EP0772470 describes a portable pump apparatus for use in CAPD is provided as well as a method for providing CAPD to a patient. The portable pump apparatus for CAPD has a housing containing a pump, and a power supply, preferably a rechargeable battery, connected to the housing and electrically connected to drive the pump.

For each of the above reasons, an improved APD machine is needed accordingly.

### SUMMARY

According to the present invention there is a provided a peritoneal dialysis ("PD") system according to claim 1.

The present disclosure sets forth a streamlined automated peritoneal dialysis APD cycler and associated system that uses a micropump. The micropumps of the cyclers of the present disclosure may be made of stainless steel and/or lower cost polymers. In one implementation, the pump head that contacts the dialysis fluid is made of plastic..

Volumetric accuracy may be derived from the pump itself. That is, each rotation of the rotor or vane, or each actuation of a piston or diaphragm, pumps a known quantity of fluid, so that the total volume of dialysis fluid pumped to or removed from the patient is the volume per stroke multiplied by the number of strokes. Certain rotating vane pumps suitable for the micropump APD cycler and associated system of the present disclosure are only accurate in one dimension.

Fresh dialysis fluid pumped by the micropump may be heated in one embodiment via an inline heater, which may be a resistance plate heater, such as a clamshell heater into which a serpentine fluid pathway of the disposable set is placed. Other types of inline heating may be used alternatively or additionally, such as radiant heating or inductive heating. Batch heaters may further alternatively be employed.

Valves used with a micropump APD cycler may be two- or three-way stopcock valves having a disposable portion that includes a stem that is rotated into multiple positions to direct fluid in a desired direction. The disposable stem fits onto a reusable actuator that rotates the stem to a desired position. In an alternative embodiment, electromechanical pinch valves may be used to occlude the lines leading to the micropump and pressure sensors. Cassette-based valves, such as pneumatic valves may be used.

The rotating positive displacement micropumps of the present disclosure, such as rotating rotor or vane pumps, produce pulsating inlet and outlet pressures may be uncomfortable to the patient. The pressure sensor, such as a pod pressure sensor, between the micropump and the patient can measure the positive pressure of fresh dialysis fluid pumped to the patient and the negative pressure of used dialysis fluid removed from the patient. The pod pressure sensor includes a pod having a flexible membrane or diaphragm that separates a dialysis fluid side of the pod from a pressure transmission fluid (e.g., air) side of the membrane. The membrane or diaphragm also serves a second purpose, namely, to dampen the pump's input and output pressure fluctuations.

Described herein is a second pressure sensor or pod, which may be placed between the micropump and the fluid source or drain. The fluid source may be multiple bags of premixed dialysis fluid or an online fluid source that mixes dialysis fluid at the point of use.

Another solution contemplated for the system and methodology of the present disclosure for managing the pulsatile nature of the micropump is to employ flowrate profiles. The peritoneal dialysis treatment of the present disclosure includes the cyclical introduction and removal of dialysis fluid to and from the patient's peritoneal cavity. Excess pressure in either direction may cause patient discomfort. The present system and associated methodology however capitalize on the phenomenon that the patient is most susceptible to discomfort at the beginning and end of both patient fills and patient drains. During the middle of both the fills and drains, patient discomfort is less likely and/or less intense. It is accordingly contemplated to employ flowrate profiles that begin a patient fill or drain at a lower flowrate, which is maintained until a certain amount of fluid is delivered to or removed from the patient, e.g., the first ten percent of a total fill or drain volume, after which the flowrate is ramped up to full speed according to a desired acceleration curve, e.g., linear or sinusoidal. At a certain point near the end of the fill or drain, e.g., the last ten percent, the flowrate is ramped down to the lower flowrate according to a desired deceleration curve, e.g., linear or sinusoidal. The system and methodology of the present disclosure may employ the flowrate profiles alone or in combination with pressure dampeners or pod pressure sensors to address the pulsatile input and output of the micropumps.

The amount of used dialysis fluid or effluent that needs to be removed from the patient after a dwell period is not known exactly because it is not known how much ultrafiltration (patient water) has osmosed into the patient's peritoneal cavity during a subsequent dwell phase, and it is not known how much residual fluid remains in the patient's cavity prior to the previous patient fill. The micropump system and method are accordingly able to detect when the patient is empty or near empty, so that a patient drain via the micropump can be terminated. The output of pressure sensor located between the patient and the micropump can be monitored. If a negative inlet pressure to the micropump increases (becomes more negative), e.g., to a threshold negative pressure or at a threshold rate of change in pressure, the control unit of the micropump APD cycler determines that the patient is empty or near empty. The negative pressure increases when the patient reaches empty because the pores in the patient's indwelling catheter begin to become blocked and a higher pressure gradient is needed to maintain flow. When the negative pressure spike is detected, the control unit of the micropump APD cycler causes the micropump to stop, completing the drain. The cycler then transitions to fill the patient with new dialysis fluid.

Peritoneal dialysis is commonly performed while the patient is sleeping, during which the patient may shift or turn and consequently fold or kink the patient line, occluding flow. The micropump system and method of the present disclosure is accordingly configured to use the pressure sensor or pod located between the micropump and the patient to look for a rise in pressure (an increase in negative pressure for a drain and an increase in positive pressure for a fill). If the pressure rises to a certain point, or a certain rate of pressure increase is detected, the patient fill or drain is stopped, and an alarm is sounded.

In light of the disclosure set forth herein, and without limiting the disclosure in any way, the present disclosure describes a peritoneal dialysis ("PD") system includes: a cycler including a micropump actuator, a pressure transducer, and at least one valve actuator; a disposable set including a micropump head sized and shaped for mating with and being cyclically driven by the micropump actuator, a pressure sensor configured to operably communicate with the pressure transducer, and at least one fluid valve portion or a portion of at least one fluid line for interfacing with the at least one valve actuator; and a control unit, wherein the disposable set is arranged to allow, and the control unit is programmed to operate the micropump actuator and the at least one valve actuator so that fresh and used dialysis fluid flows through the micropump head in a same direction.

The micropump actuator and the micropump head may be more accurate in the same direction than in a reverse direction.

The micropump actuator may include a keyed driver and the micropump head includes a keyed recess sized and shaped for mating with the keyed driver of the micropump actuator.

The pressure sensor may include a fluid pressure chamber having a sealed diaphragm configured to fluctuate due to pressure variations, and wherein the cycler includes a transmission fluid chamber positioned and arranged to mate with the fluid pressure chamber when the disposable set is mounted to the cycler.

A transmission fluid line may extend from the transmission fluid chamber to the pressure transducer.

The pressure sensor may include a fluid pressure chamber having a sealed diaphragm configured to fluctuate due to pressure variations, and a transmission fluid chamber sealed to the fluid pressure chamber.

The pressure sensor may interface with the pressure transducer via a transmission fluid line placed in fluid communication with the transmission fluid chamber.

At least one fluid valve portion may include a rotating stem and the at least one valve actuator is configured to rotate the rotating stem.

The valve actuator may be a pinch valve positioned and arranged to occlude the portion of at least one fluid line when the disposable set is mounted to the cycler.

Described is a disposable set including an inline fluid heating pathway or a batch heater container, and the cycler includes an inline fluid heater or batch fluid heater positioned and arranged to heat the inline fluid heating pathway or the batch heater container, respectively, when the disposable set is mounted to the cycler.

The disposable set may be arranged to allow fresh and used dialysis fluid to flow through the micropump head in a same direction includes disposing both a patient line and a drain line of the disposable set, at least during a patient fill phase, downstream of the micropump head.

The control unit may be programmed to operate the micropump actuator so that at least one of fresh or used dialysis fluid flows at a lower flowrate at a beginning of at least one of a patient fill or a patient drain, respectively, than during a middle portion of the at least one of the patient fill or the patient drain.

The control unit may be further programmed to operate the micropump actuator so that the at least one of fresh or used dialysis fluid flows at a lower flowrate at an end of the at least one of the patient fill or the patient drain, respectively, than during a middle portion of the at least one of the patient fill or the patient drain.

Described herein is a disposable set further including at least one pressure sensor positioned and arranged to dampen pressure fluctuations of fresh or used dialysis fluid flowing between the micropump head and a patient line.

The control unit may be programmed to monitor a negative pressure from the pressure transducer during a patient drain in which the micropump actuator actuates the micropump head to remove used dialysis fluid from a patient, and wherein if the sensed negative pressure increases to a predefined extent, the control unit ends the patient drain.

The predefined extent may include (i) a threshold value, (ii) a threshold rate of change of negative pressure, or (iii) a combination of (i) and (ii).

Described herein is a peritoneal dialysis ("PD") system includes: a cycler including a micropump actuator, and a pressure transducer; a disposable set including a micropump head sized and shaped for mating with and being cyclically driven by the micropump actuator, and a pressure sensor configured to operably communicate with the pressure transducer; and a control unit programmed to monitor a negative pressure from the pressure transducer and a drain flowrate during a patient drain in which the micropump actuator actuates the micropump head to remove used dialysis fluid from a patient, and wherein if the sensed negative pressure increases to a first predefined extent and/or if the drain flowrate falls to a second predefined extent, the control unit determines that a drain occlusion has occurred.

Described herein the first predefined extent includes (i) a threshold value, (ii) a threshold rate of change of negative pressure, or (iii) a combination of (i) and (ii).

Described herein the second predefined extent includes (i) a threshold value, (ii) a threshold rate of change of flowrate, (iii) a combination of (i) and (ii), or (iv) a combination of (i), (ii) or (iii) with an output from a flowrate sensor.

Described herein, the control unit upon determining that the drain occlusion has occurred causes an instruction to be provided for clearing the occlusion.

Described herein, a peritoneal dialysis ("PD") system includes: a cycler including a micropump actuator, and a pressure transducer; a disposable set including a micropump head sized and shaped for mating with and being cyclically driven by the micropump actuator, and a pressure sensor configured to operably communicate with the pressure transducer; and a control unit programmed to monitor a positive pressure from the pressure transducer and a fill flowrate during a patient fill in which the micropump actuator actuates the micropump head to deliver fresh dialysis fluid to a patient, and wherein if the sensed positive pressure increases to a first predefined extent and/or if the fill flowrate falls to a second predefined extent, the control unit determines that a fill occlusion has occurred.

Described herein, the first predefined extent includes (i) a threshold value, (ii) a threshold rate of change of positive pressure, or (iii) a combination of (i) and (ii).

Described herein, the second predefined extent includes (i) a threshold value, (ii) a threshold rate of change of flowrate, (iii) a combination of (i) and (ii), or (iv) a combination of (i), (ii) or (iii) with an output from a flowrate sensor.

Described herein, the control unit upon determining that the fill occlusion has occurred causes an instruction to be provided for clearing the occlusion.

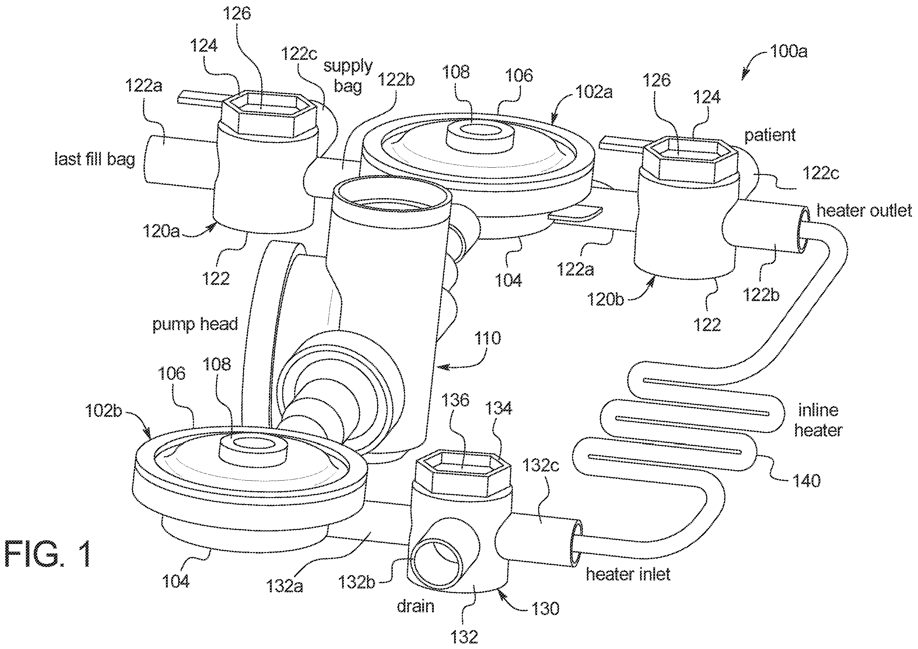

Any of the features, functionality and alternatives described in connection with any one or more of Figs. 1 to 13B may be combined with any of the features, functionality and alternatives described in connection with any other of Figs. 1 to 13B.

It is accordingly an advantage of the present disclosure to provide a micropump driven APD system, which is portable to ultra-portable (e.g., wearable) and compact.

It is another advantage of the present disclosure to provide a micropump driven APD system, wherein the micropump is at least partially reusable.

It is a further advantage of the present disclosure to provide a micropump driven APD system, which eliminates certain sealing issues present in known APD systems.

It is yet a further advantage of the present disclosure to provide a micropump driven APD system, which eliminates bulky pneumatic equipment associated with certain APD systems.

It is yet another advantage of the present disclosure to provide a micropump driven APD system, which manages peritoneal dialysis fluid flow so as to be within safe and comfortable patient pressure limits.

It is still another advantage of the present disclosure to provide a micropump driven APD system, which may reduce time to detect a patient empty condition and thus overall treatment time.

It is still a further advantage of the present disclosure to provide a micropump driven APD system, which may reduce patient exposure time to lower pressure.

Further still, it is an advantage of the present disclosure to provide a micropump driven APD system that can maintain flowrates under partial occlusion and thereby maintain or improve treatment time as compared with other APD systems.

Moreover, it is an advantage of the present disclosure to provide a portable APD system, wherein solution bag size may be reduced for treatment on the go.

Additional features and advantages are described in, and will be apparent from, the following Detailed Description and the Figures..

### BRIEF DESCRIPTION OF THE FIGURES

Fig. 1 is a perspective view from the bottom of a disposable set for the micropump automated peritoneal dialysis ("APD") cycler of the present disclosure, wherein the disposable set uses stopcock valves.
Fig. 2 is an enlarged view of a disposable, fluid-contacting micropump head of the present disclosure provided with the disposable set of Fig 1.
Fig. 3 is a perspective view of a micropump APD cycler of the present disclosure operating the disposable set of Fig 1.
Fig. 4 is a plan view of another disposable set for the APD cycler of the present disclosure, wherein the disposable set is configured for operation with multiple three-way valves.
Fig. 5 is a plan view of a further disposable set for the APD cycler of the present disclosure, wherein the disposable set is configured for operation with pinch valves for tubing occlusion.
Fig. 6 is a plan view of another disposable set for the APD cycler of the present disclosure, wherein the disposable set is configured for operation with batch heating and a micropump that is accurate in two directions.
Fig. 7A is a schematic view illustrating a patient fill using a micropump of the present disclosure.
Fig. 7B is a schematic view illustrating a patient drain using a micropump of the present disclosure.
Fig. 8 is a plot illustrating one patient empty detection using the micropump APD system and method of the present disclosure.
Fig. 9 is a plot illustrating one drain occlusion detection using the micropump APD system and method of the present disclosure.
Fig. 10 is a plot illustrating one fill occlusion detection using the micropump APD system and method of the present disclosure.
Figs. 11A and 11B are plots illustrating the use of the micropump APD system and method of the present disclosure, which show the effects of pressure pod dampening for a larger patient fill flowrate.
Figs. 12A and 12B are plots illustrating the use of the micropump APD system and method of the present disclosure, which show the effects of pressure pod dampening for a low patient fill flowrate.
Figs. 13A and 13B are plots illustrating the use of the micropump APD system and method of the present disclosure, which show the effects of pressure pod dampening for a larger patient drain flowrate.

### DETAILED DESCRIPTION

Referring now to the drawings and in particular to Fig. 1, a disposable set 100a having a disposable, fluid-contacting micropump head 110 is illustrated. Fluid-contacting micropump head 110 is located between first and second disposable, pressure sensors or pods 102a and 102b. Pressure sensor or pod 102a is located fluidly between first and second three-way disposable, fluid-contacting stopcock valve portions 120a and 120b. Pressure sensor 102b or pod is located fluidly between fluid-contacting micropump head 110 and a three-way disposable, fluid-contacting stopcock valve portion 130.

Disposable set 100a includes an inline fluid heating pathway 140. Inline fluid heating pathway 140 is located fluidly between first and second disposable, pressure sensors 102a and 102b and valve portions 120b and 130. Suitable materials for any of disposable set 100a, including any of pressure sensors 102a and 102b, fluid-contacting micropump head 110, fluid-contacting stopcock valve portions 120a, 120b and 130, inline fluid heating pathway 140 and any associated tubing include polyvinyl chloride ("PVC"), polyethylene ("PE"), polyurethane ("PU"), polycarbonate or other non-PVC material.

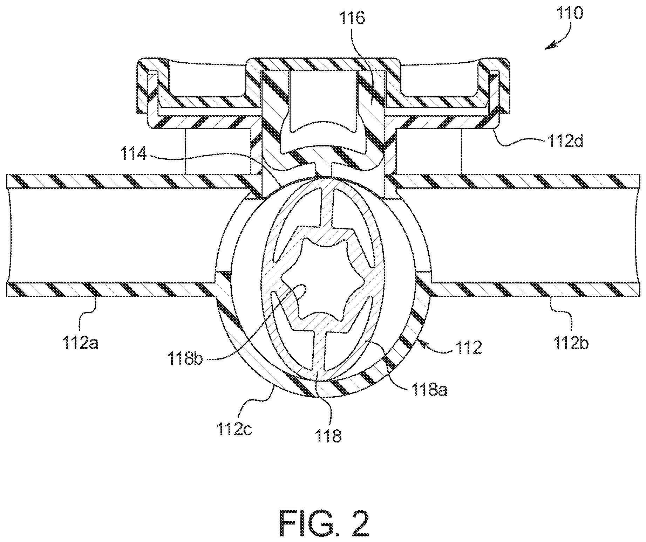

Disposable set 100a is illustrated from the bottom to show how different disposable components interact with their corresponding actuators. The underside of fluid-contacting micropump head 110 is illustrated in more detail in connection with Fig. 2. The micropump including micropump head 110 may be provided for example by Quantex, London, W6 7HJ, UK. Fig. 2 illustrates that fluid-contacting micropump head 110 includes a housing 112 including or defining an inlet port 112a, an outlet port 112b, a rotor holding section 112c in fluid communication with the inlet port 112a and outlet port 112b, and a plunger holding section 112d that does not communicate fluidly with ports 112a, 112b or rotor holding section 112c. In an alternative, plunger holding section 112d may communicate fluidly with outlet port 112b. A thin membrane 114 separates rotor holding section 112c from membrane holding section 112d. A spring-loaded plunger 116 is fitted into membrane holding section 112d and applies a desired amount of holding force to membrane 114, which may be attached to housing 112 or be molded as a thin portion of housing 112, and therefore be of the same material as housing 112.

The holding force maintains membrane so as to be in contact with a rotor 118 over a full revolution and for each revolution. Rotor 118 includes an elliptical or oblong fluid contacting driver 118a that moves fresh or used dialysis fluid from inlet port 112a to outlet port 112b. Rotor 118 also includes or defines a keyed recess 118b, which in illustration has the shape of a star. Keyed recess 118b fits onto a reusable micropump actuator, which is located within a cycler or machine of the present disclosure. The micropump actuator is operated under control of a control unit to spin at a desired or preprogrammed rate. The rate is variable for reasons discussed herein.

Fluid-contacting micropump head 110 may be operated in two directions, such that the roles of inlet port 112a and outlet port 112b may be reversed. Certain types of micropumps are only accurate in one direction, so that at least some of the flowpaths discussed below only route fresh or used dialysis fluid in one direction through micropump head 110, at least where accuracy is required. Regarding accuracy, in Fig. 2, fluid-contacting micropump head 110 is constructed such that one full rotation of rotor 118 pumps a known amount of fresh or used dialysis fluid. The control unit knows how many revolutions the micropump actuator makes over a fill or drain phase, for example, by providing a motor encoder with the motor of the micropump actuator, which outputs the number of revolutions and partial revolutions made to the control unit. The control unit then multiplies the number of full and partial revolutions by the known volume per revolution to determine the amount of fresh or used dialysis fluid delivered on a real time basis. Volumetric accuracy for fluid-contacting micropump head 110 of Fig. 2 has been demonstrated to be ≤ ±2 % for a flowrate range of 35 mL/min to 350 mL/min. The number of full and partial revolutions divided by the time period over which the revolutions have occurred determines dialysis fluid flowrate, which the control unit controls, for example, by controlling the current provided to the motor of the micropump actuator. Example ranges for revolutions per second ("rps") for micropump head 110 to achieve the flowrate range of 35 mL/min to 350 mL/min are 1 rps to 10.4 rps.

Further information regarding the micropump of Fig. 2 may be found in U.S. Patent No. 10,087,931. The micropumps of the present disclosure are, however, not limited to the type illustrated in connection with Fig. 2. For example, the micropumps may instead be piston and cylinder pumps, e.g., a Swissinova Tpump or a Swissinova Nao Stedi pump described in U.S. Patent Publication No. 2015/00147210. Such piston and cylinder pumps likewise have reusable and a disposable components and control volume accurately by enabling known volume pump strokes to be summed.

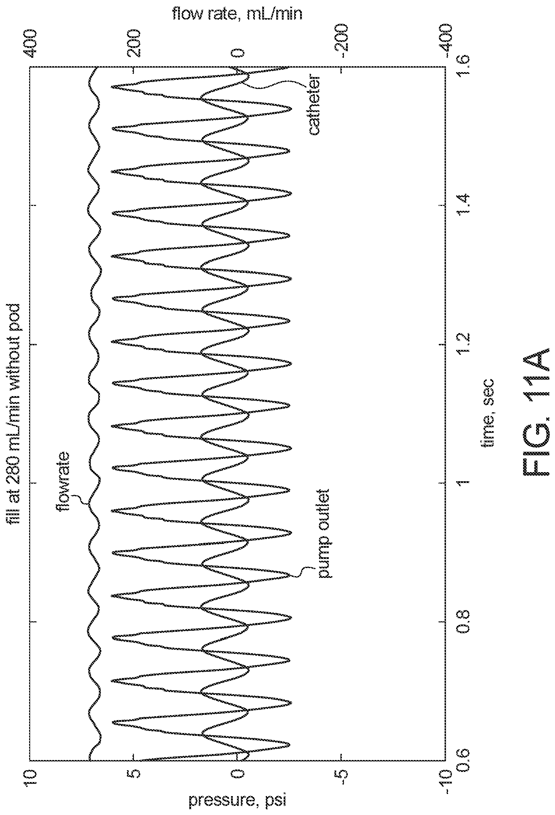

The micropump of Fig. 2 including fluid-contacting micropump head 110 is a relatively continuous pump because it pulls dialysis fluid on the suction side of housing 112 and rotor 118 as rotor 118 pushes dialysis fluid out of housing 112. Over a full stroke, however, fluid-contacting micropump head 110 is pulsatile as the per-stroke flowrate varies depending on the location fluid contacting driver 118a over a revolution. It is accordingly contemplated to place a pressure sensor 102a and 102b at least one of upstream and/or downstream of fluid-contacting micropump head 110. Pressure sensors 102a and 102b and their associated pressure transducers output to the control unit measure a positive (e.g., to the patient) or negative (e.g., from the patient) dialysis fluid pumping pressure. Pressure sensors or pods 102a and 102b each include a pressure transmitting diaphragm, which dampens pressure pulses and smoothens downstream fluid flow as illustrated below in connection with Figs. 11A, 11B, 12A, 12B, 13A and 13B.

In particular, Fig. 1 illustrates that pressure sensors 102a and 102b each include a dialysis fluid pressure chamber 104 and a transmission fluid (e.g., air) chamber 106 that seal together about a pressure transmitting diaphragm (not seen in Fig. 1). Transmission fluid chamber 106 includes or defines a transmission line port 108 that accepts a transmission line (not illustrated), which extends to a pressure transducer located within the cycler of the present system and method. Positive or negative fluid pressure within dialysis fluid pressure chamber 104 is transmitted via the diaphragm to the transmission fluid or air within transmission fluid chamber 106 and the transmission line to pressure transducer, which outputs a corresponding pressure signal to the control unit of the APD cycler. In Fig. 1, dialysis fluid pressure chamber 104 and the pressure diaphragm are disposable, e.g., single use disposable. The pressure transmission line to pressure transducer is reusable. Transmission fluid chamber 106 may be reusable or disposable, that is, when installing disposable set 100a for operation with the cycler, either (i) dialysis fluid pressure chamber 104 becomes removably sealed to transmission fluid chamber 106 or (ii) the transmission fluid line becomes removably sealed to transmission line port 108 of transmission fluid chamber 106. In an alternative implementation of either (i) or (ii) above, the pressure transmission line is not provided and transmission fluid chamber 106 instead couples directly to the pressure transducer for operation.

Fig. 1 also illustrates that three-way fluid-contacting stopcock valve portions 120a and 120b each include a valve housing 122 and associated inlet and outlet ports 122a, 122b and 122c. A valve stem 124 is rotationally sealed within valve housing 122 and is configured to allow or disallow fresh or used dialysis fluid flow between inlet and outlet ports 122a, 122b and 122c through housing 122, depending on the rotational position of valve stem 124. Valve stem 124 includes an open and a closed position. Valve stem 124 includes or defines a keyed recess 126, which as illustrated has the shape of a hexagon. Keyed recess 126 fits onto a reusable three-way valve actuator, which is located within the cycler or machine of the present disclosure. The three-way actuators associated with fluid-contacting stopcock valve portions 120a and 120b are operated under control of the control unit to rotate valve stem 124 to the open (allow flow) or closed (no flow) position.

Fig. 1 also illustrates that three-way fluid-contacting stopcock valve portion 130 includes a valve housing 132 and associated inlet and outlet ports 132a, 132b and 132c. A valve stem 134 is rotationally sealed within valve housing 132 and is configured to allow or disallow fresh or used dialysis fluid flow between (i) inlet and outlet ports 132a and 132b, (ii) inlet and outlet ports 132a and 132c, and (iii) inlet and outlet ports 132b and 132c (e.g., in case inline fluid heating pathway 140 needs to be drained), and (iv) total occlusion of flow through housing 132, depending on the rotational position of valve stem 134. Valve stem 134 includes two open positions and a closed position. Valve stem 134, like stem 124, includes or defines a keyed recess 136, which as illustrated also has the shape of a hexagon. Keyed recess 136 fits onto a reusable three-way valve actuator, which is located within the cycler or machine of the present disclosure. The three-way actuator associated with fluid-contacting stopcock valve portions 130 is operated under control of the control unit to rotate valve stem 134 to (i) a first open position (allow between ports 132a and 132b), (i) a second open position (allow between ports 132a and 132c) or (iii) a closed (no flow) position. In general, any of the ports for each of three-way valves 120a, 120b and 130 can be fluidly communicated with any other of the three ports, e.g., during final emptying of disposable set 100a at the end of treatment.

In the illustration, tubing attached to port 122a of three-way fluid-contacting stopcock valve portion 120a extends to a last fill container or bag, which may for example contain icodextrin, which has a different formulation than the peritoneal dialysis solution used in prior fills. Tubing attached to port 122c of three-way fluid-contacting stopcock valve portion 120a extends to a supply container or bag of peritoneal dialysis solution used in the prior fills. Tubing attached to port 122c of three-way fluid-contacting stopcock valve portion 120b extends to the patient. Tubing attached to port 122b of three-way fluid-contacting stopcock valve portion 120b extends to an inline heater outlet lines. Tubing attached to port 132c of three-way fluid-contacting stopcock valve portion 130 extends to the inline heater inlet line. Tubing attached to port 132b of three-way fluid-contacting stopcock valve portion 130a extends to a drain container or bag or to a house drain.

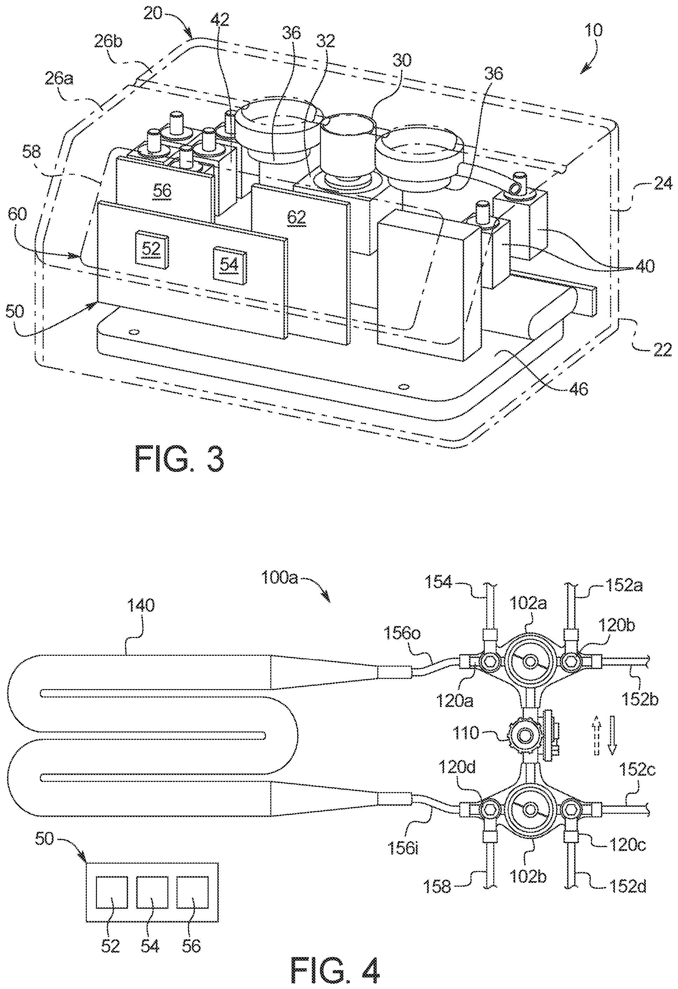

Fig. 3 illustrates one possible operational mounting arrangement for disposable set 100a. Here, system 10 includes an APD cycler or machine 20. APD cycler 20 includes a housing 22 having a base 24 and lids 26a and 26b hingedly connected to base. Housing 22 may be made of metal, such as stainless steel, steel, aluminum and combinations thereof and/or a plastic, such as, any one or more of polyvinyl chloride ("PVC"), polyethylene ("PE"), polyurethane ("PU"), or polycarbonate. Base houses a control unit 50, which includes one or more processor 52, one or more memory 54 and a video controller 56 for controlling a video monitor 58. Video monitor 58 is part of an overall user interface 60 for systems 10 described herein. User interface 60 includes any one or more of a touch screen overlay operable with video monitor 58 and/or one or more electromechanical input device, e.g., membrane switches, for inputting information into control unit. Video monitor 58 and speakers (e.g., operable with a sound card 62 of control unit 50) are provided to output information to the patient or user, e.g., alarms, alerts and/or voice guidance commands. Control unit 50 may also include a transceiver and a wired or wireless connection to a network, e.g., the internet, for sending treatment data to and receiving prescription instructions from a doctor's or clinician's server interfacing with a doctor's or clinician's computer.

Lids 26a and 26b open to allow disposable set 100a to be translated down into APD cycler 20 so that keyed recess 118b of rotor 118 of fluid-contacting micropump head 110 fits onto a mating keyed driver of reusable micropump actuator 30. Reusable micropump actuator 30 is driven by a motor 32 under control of control unit 50, such as a stepper motor, which may be fitted with an encoder that outputs to the control unit.

In Fig. 3 regarding pressure sensors or pods 102a and 102b, either (i) dialysis fluid pressure chamber 104 becomes removably sealed to transmission fluid chamber 106 located within cycler base 24 and reusable or (ii) the transmission fluid line located within cycler base 24 becomes removably sealed to transmission line port 108 of transmission fluid chamber 106 (here disposable). In an alternative implementation of either (i) or (ii) above, the pressure transmission line is not provided and transmission fluid chamber 106 instead couples directly to the pressure transducer 36 (located within cycler base 24) for operation. In an embodiment, a separate pressure transducer 36 outputting to control unit 50 is provided for each pressure sensor 102a and 102b

In Fig. 3, keyed recesses 126 of valve stems 124 of three-way fluid-contacting stopcock valve portions 120a and 120b fit onto a mating reusable three-way valve actuators 40 located within cycler base 24. Keyed recess 136 of valve stem 134 of three-way fluid-contacting stopcock valve portion 130 fits onto a mating reusable three-way valve actuator 42 located within cycler base 24. Valve actuators 40 and 42 are under the control of control unit 50 and operate as described above to selectively allow or disallow fresh or used dialysis fluid flow in desired directions.

In Fig. 3, inline fluid heating pathway 140 of Fig. 1 is located within an inline fluid heater 46 located with cycler base 24. Inline fluid heater 46 is under control of control unit 50 and may be a resistance plate heater, such as a clamshell heater into which a serpentine pathway, for example, of heating pathway 140 is placed. Other types of inline heating may be used alternatively or additionally, such as radiant heating and/or inductive heating. Heater 46 may further alternatively be a batch heater. Here, the heating portion of the disposable set is a bag or pouch that receives fresh dialysis fluid for heating. In Fig. 3, inline fluid heater 46 is located near the bottom of cycler base 24. As an alternative, inline fluid heater 46 may be located at the back or one of the sides of cycler base 24.

Referring now to Fig. 4, an alternative implementation of disposable set 100a is illustrated using four three-way disposable, fluid-contacting stopcock valve portions 120a to 120d instead of two three-way valve portions and a single three-way disposable, fluid-contacting stopcock valve portion 130. Fluid-contacting micropump head 110 is provided again with first and second disposable, pressure sensors 102a and 102b. In the illustration, three-way fluid valve portions 120b and 120c are selectively fluidly connected to supply lines 152a to 152c, which are in turn connected to fresh dialysis fluid supply containers (e.g., bags) and a last fill line 152d, which is connected to a last fill dialysis fluid supply container (e.g., bag, last fill for extended dwell). Three-way fluid valve portion 120a is alternatively fluidly connected to a patient line 154 leading to the patient or a heater outlet line 156o leading to an exit of inline serpentine heating pathway 140. Three-way fluid valve portion 120d is alternatively fluidly connected to a drain line 158 leading to a drain container (e.g., bag) or house drain (e.g., toilet or bathtub) or a heater inlet line 156i leading to an inlet of inline serpentine heating pathway 140.

In Fig. 4, for a patient fill, fresh dialysis fluid flows from one of supply lines 152a to 152c or last fill line 152d, through inlet pressure sensor 102a, micropump head 110, outlet pressure sensor 102b, heater inlet line 156i, serpentine heating pathway 140, heater outlet line 156o, to patient line 154 and the patient. For upper supply lines 152a and 152b, micropump head 110 pushes fresh dialysis fluid through serpentine heating pathway 140 to the patient via patient line 154.

For lower supply and last fill lines 152c and 152d, micropump head 110 is run in reverse to pull fresh dialysis fluid from a supply container connected to lines 152c and 152d (one at a time) to fill empty supply container or bags connected to lines 152a or 152b, after which fresh dialysis fluid is pumped to the patient via serpentine heating pathway 140 via patient line 154. This procedure ensures that the accurate pump direction is used to deliver fluid to patient via the inline heater regardless of which supply container or bag is used. Container or bags 152a and 152b are emptied to drain prior to bags 152c and 152d being needed for treatment. Reverse pumping for micropump head 110, although not accurate across flow and pressure boundary conditions, is nevertheless available for pumping in situations in which flow and pressure accuracy is not important. When pumping in the accurate direction using micropump head 110, a signal from outlet pressure sensor 102b to control unit 50 is used as feedback to ensure that fresh dialysis fluid positive pressure to the patient is at or within a limit, e.g., 20.6 kPa (3 psig) out of a range of 10.3 to 62kPa (1.5 psig to 9 psig).

In Fig. 4, for a patient drain, used dialysis fluid is pulled from the patient, though patient line 154, inlet pressure sensor 102a, micropump head 110, outlet pressure sensor 102b, and drain line 158 leading to a drain container or house drain. The signal from inlet pressure sensor 102a to control unit 50 is used as feedback to ensure that used dialysis fluid negative pressure to pull from the patient is at or within a limit, e.g., -10.3kPa (-1.5 psig).

It should be appreciated that for both patient fills and drains system 10 in connection with Fig. 4, fresh and used dialysis fluid flows through micropump head 110 in the same direction, which allows for the micropump to only have to be accurate in one direction. The micropump including micropump head 110 can pump in either direction, e.g., for bag replenishment as discussed above and in response to an occlusion as discussed below. As mentioned above, reverse pumping may be performed where a high amount of accuracy is not required. It should be appreciated that micropump head 110 as illustrated in detail in connection with Fig. 2 may be replaced in the system 10 of Fig. 4 with the piston and cylinder micropump, e.g., the Swissinova Tpump or the Swissinova Nao Stedi pump discussed above, which are likewise operated under control of control unit 50.

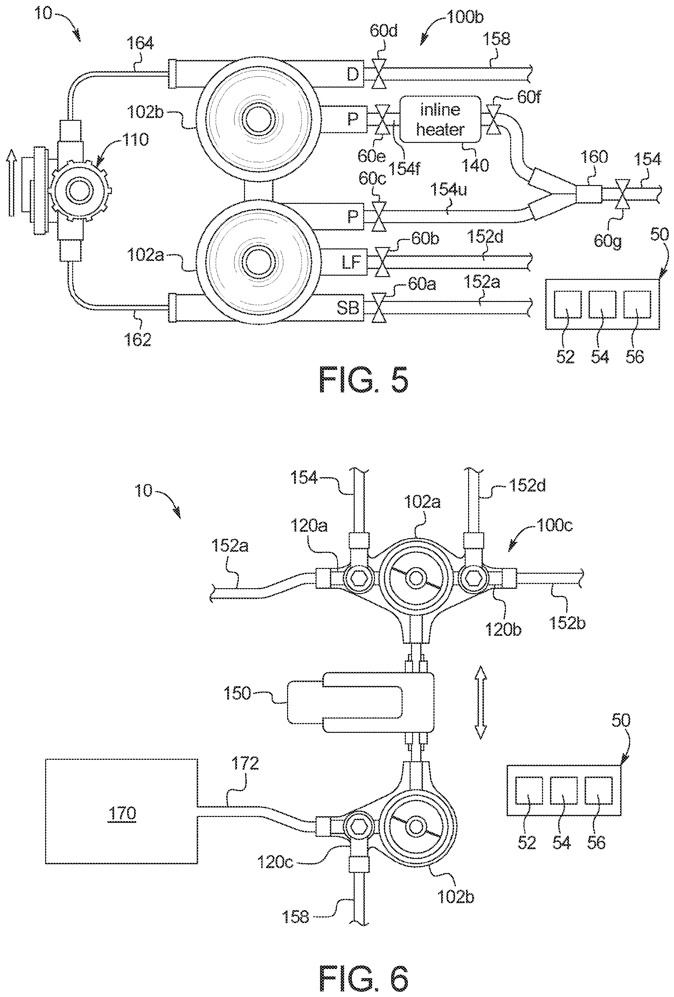

Referring now to Fig. 5, an alternative disposable set 100b for use with system 10 is illustrated. Disposable set 100b may be mounted for operation to cycler 20 in any of the manners discussed above in connection with Fig. 3. The primary difference between disposable set 100b and disposable set 100a is that disposable set 100b is configured to operate with electromechanical pinch valves 60a to 60g as opposed to the stopcock valves described above. Electromechanical pinch valves 60a to 60g operating under control of control unit may be motorized pinch valves or solenoid pinch valves, either of which may fail closed upon loss of power, so that all fluid lines close safely. Electromechanical pinch valves 60a to 60g are reusable and located within cycler 20.

Disposable set 100b includes fluid-contacting micropump head 110 and first and second disposable, pressure sensors or pods 102a and 102b, including all of their structure, functionality and alternatives described herein. Pressure sensor 102a is again an inlet pressure sensor that monitors a negative incoming fresh dialysis fluid pressure from supply line 152a (connected fluidly to a supply container), last fill line 152d (connected fluidly to a last fill container) and a negative incoming used dialysis fluid pressure from a used fluid patient line 154u. Pressure sensor 102b is again an outlet pressure sensor that monitors a positive outgoing fresh dialysis fluid pressure through fresh fluid patient line 154f and a positive outgoing used dialysis fluid pressure through drain line 158.

In the illustration, disposable set 100b provides a Y- or T-connector 160 that fluidly merges fresh patient line 154f and used patient line 154u into a common patient line 154 that leads to the patient's catheter set. Inline fluid heating pathway 140 is illustrated in block form as being located along fresh patient line 154f. Pinch valve 60a selectively opens or occludes supply line 152a. Pinch valve 60b selectively opens or occludes last fill line 152d. Pinch valve 60c selectively opens or occludes used fluid patient line 154u. Pinch valve 60d selectively opens or occludes drain line 158. Pinch valve 60e selectively opens or occludes fresh fluid patient line 154f upstream from fluid heating pathway 140. Pinch valve 60f selectively opens or occludes fresh fluid patient line 154f downstream from fluid heating pathway 140. Pinch valve 60g selectively opens or occludes common patient line 154.

Fluid-contacting micropump head 110 communicates fluidly with (i) the outlet from pressure sensor 102a via pump inlet line 162 and (ii) the inlet to pressure sensor 102b via pump outlet line 164. In Fig. 5, for a patient fill, fresh dialysis fluid flows from supply lines 152 or last fill line 152d, through inlet pressure sensor 102a, pump inlet line 162, micropump head 110, pump outlet line 164, outlet pressure sensor 102b, fresh fluid patient line 154f and serpentine heating pathway 140, Y- or T-connector 160 and common patient line 154 to the patient. Here, one of pinch valve 60a or 60b is open and valves 60e to 60g are open, while the other one of valves 60a or 60b, valve 60c and drain valve 60d are closed. The signal from outlet pressure sensor 102b to control unit 50 is used as feedback to ensure that fresh dialysis fluid positive pressure to the patient is at or within a limit, e.g., 20.6 kPa (3 psig).

In Fig. 5, for a patient drain, used dialysis fluid is pulled from the patient, through common patient line 154, Y- or T-connector 160, used fluid patient line 154u, inlet pressure sensor 102a, pump inlet line 162, micropump head 110, pump outlet line 164, outlet pressure sensor 102b, and drain line 158 leading to a drain container or house drain. The signal from inlet pressure sensor 102a to control unit 50 is used as feedback to ensure that used dialysis fluid negative pressure to pull from the patient is at or within a limit, e.g., - 10.3kPa (-1.5 psig).

It should be appreciated that for both patient fills and drains of system 10 in connection with Fig. 5, fresh and used dialysis fluid flows through micropump head 110 in the same direction, which again allows for the micropump (which can pump in both directions) to only have to be accurate in one direction. It should be appreciated that micropump head 110 as illustrated in detail in connection with Fig. 2 may be replaced in the system 10 of Fig. 5 with the piston and cylinder micropump, e.g., the Swissinova Tpump or the Swissinova Nao Stedi pump, discussed above, which are likewise operated under control of control unit 50.

Referring now to Fig. 6, another alternative disposable set 100c for use with system 10 is illustrated. Disposable set 100c may be mounted for operation to cycler 20 in any of the manners discussed above in connection with Fig. 3. The primary difference between disposable set 100c and disposable set 100a is that disposable set 100c is configured to operate with a heater container or bag 170 via a heater bag line 172 for batch heating instead of inline heating. Another difference is that the micropump having an alternative fluid-contacting micropump head 150 pumps accurately in two directions, and may be a piston and cylinder type micropump, e.g., Swissinova Tpump, discussed above, operated under control of control unit 50. It should be appreciated that the batch heating of the present disclosure does not require pumping accuracy in two directions, rather, the example disposable set 100c of Fig. 6 illustrates that system may be operated with such capability.

Fluid-contacting micropump head 150 is located between first and second disposable, pressure sensors or pods 102a and 102b. Pressure sensor 102a is located fluidly between first and second three-way disposable, fluid-contacting stopcock valve portions 120a and 120b. Pressure sensor 102b is in fluid communication with a third three-way disposable, fluid-contacting stopcock valve portion 120c. Three-way valve portion 120a provides alternative fluid communication with the patient via patient line 154 or a first supply line 152a leading to a first supply container, e.g., bag. Three-way valve portion 120b provides alternative fluid communication with a second supply line 152b leading to a second supply container, e.g., bag, or a last fill line 152d, which is connected to a last fill dialysis fluid supply container (e.g., bag, last fill for extended dwell). Three-way valve portion 120c provides alternative fluid communication with heater bag 170 via a heater bag line 172 or drain line 158 leading to a drain container (e.g., bag) or house drain (e.g., toilet or bathtub).

Using disposable set 100c, control unit 50 of system 10 for a patient fill manipulates three-way valve portions 120a and 120b and actuates micropump head 150 so as to draw fresh dialysis fluid or last fill fluid from its respective source and push same to heater bag 170, where a resistive plate and/or radiant or other heater heats the batch of fresh dialysis fluid to body temperature, e.g., 37°C. Once heated, control unit 50 manipulates three-way valve portions 120a to 120c and actuates micropump head 150 so as to draw fresh, heated dialysis fluid from heater bag 170 and push same in the reverse direction to the patient via patient line 154. While it is beneficial to be accurate in both directions so as not to overfill or underfill heater bag 170, it is more important to be accurate pumping in the second direction to provide a prescribed fill volume to the patient.

During a patient dwell phase using disposable set 100c, control unit 50 causes a second batch of dialysis fluid to be heated in heater bag 170 via the sequence described above.

Using disposable set 100c, control unit 50 of system 10 for a patient drain manipulates three-way valve portions 120a and 120c and actuates micropump head 150 so as to draw used dialysis fluid from the patient via patient line 154 and push same to a drain container or house drain via drain line 158. It is important in the patient drain to be accurate to know how much effluent has been removed from the patient, so that an accurate amount of ultrafiltration ("UF") removed from the patient is calculated by control unit subtracting the fill volume from the drain volume.

One possible way to use a micropump that is accurate in one direction with batch heating is to move drain line 158 and the drain to one of supply lines 152a or 152b and make the remaining supply container larger. The now empty port of three-way valve portions 120c is then connected to an empty sample container, which initially receives a patient drain from a non-accurate flow direction. Next, control unit 50 causes a second batch of heated, fresh dialysis fluid to be pumped in an accurate direction to the patient via patient line 154. After the second patient fill, control unit 50 causes the drain fluid to be pumped from the sample container in the accurate direction to drain via drain line 158. After the removal of effluent to drain, control unit 50 causes a next patient fill volume, e.g., last fill volume, to be pumped to heater bag 170 for a subsequent patient fill.

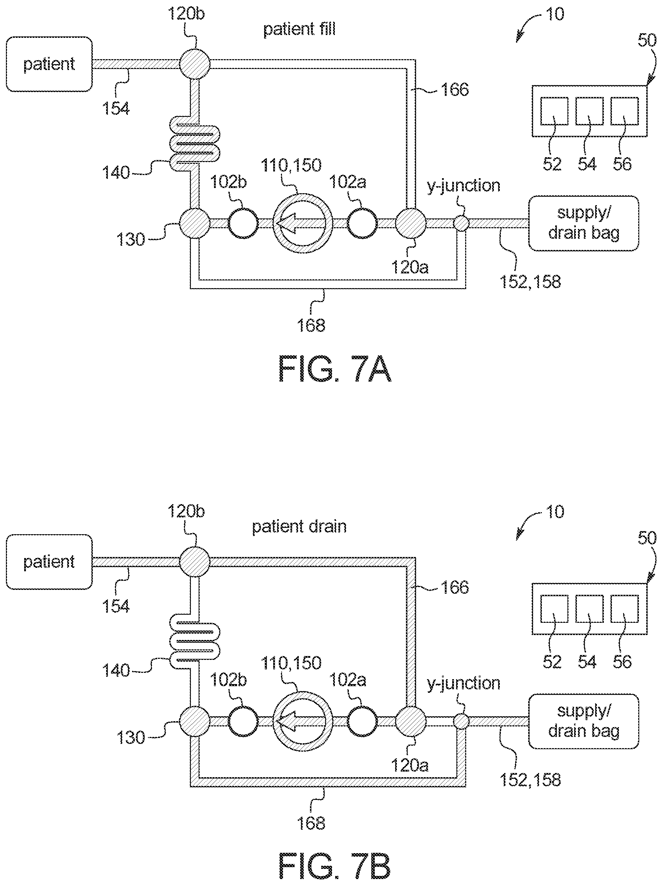

Referring now to Figs. 7A and 7B, one possible patient fill flow regime and patient drain regime, respectively, for a disposable set, such as set 100a, having a combination of three-way disposable, fluid-contacting stopcock valve portions 120a and 120b and a three-way disposable, fluid-contacting stopcock valve portion 130 is illustrated. Inlet and outlet pressure sensors or pods 102a and 102b located on either side of micropump having disposable, fluid-contacting micropump head 110 or 150 are also provided. Inline fluid heating pathway 140 in fresh dialysis fluid line is also provided. For convenience, the supply and drain containers are illustrated as the same containers communicating with common supply and drain line 152, 158, which is contemplated to reduce disposable waste and cost. The supply and drain containers are alternatively separate. Patient line 154 leads to the patient. Additional lines 166 and 168 used for the patient drain are also provided.

In Fig. 7A using disposable set 100a, control unit 50 of system 10 for a patient fill manipulates three-way valve portions 120a and 120b and three-way valve portion 130 and actuates micropump head 110 or 150 so as to draw fresh dialysis fluid or last fill fluid from its respective source via common supply and drain line 152, 158 and push same through inline fluid heating pathway 140, where a resistive plate and/or radiant or other heater heats the fresh dialysis fluid to body temperature, e.g., 37°C. The inline flow of now heated fresh dialysis then flows to the patient via patient line 154. Control unit 50 uses the signal from pressure sensor 102b to ensure that fresh dialysis fluid positive pressure to the patient is at or within a limit, e.g., 20.6kPa (3 psig).

Regarding the inline heating of system 10, preliminary data have shown positive results. The data demonstrates the flow profile from micropump head 110 or 150 in combination with the dampening discussed herein is suitable, e.g., continuous enough, to be used with inline heating and to deliver fresh, heated dialysis fluid to the patient in an accurate manner.

In Fig. 7B using disposable set 100c, control unit 50 of system 10 for a patient drain manipulates three-way valve portions 120a and 120b and three-way valve portion 130 and actuates micropump head 110 or 150 so as to draw used dialysis fluid from the patient via patient line 154 and line 166 and push same through line 168 and common supply and drain line 152, 158 to drain container or bag 152, 158. Control unit 50 uses the signal from pressure sensor 102a to ensure that used dialysis fluid negative pressure to pull effluent from the patient is at or within a limit, e.g., -10.3kPa (-1.5 psig).

It should be appreciated that for both patient fills and drains in the system 10 in connection with Figs. 7A and 7B, fresh and used dialysis fluid flows through micropump head 110 or 150 in the same direction, which allows for the micropump to only have to be accurate in one direction.

As discussed above and as illustrated below in connection with Figs. 11A, 11B, 12A, 12B, 13A and 13B, pressure sensors or pods 102a and 102b have been found to dampen the pulsatility of the micropumps of the present disclosure. Another solution contemplated for system 10 and associated methodology of the present disclosure for alternatively or additionally managing the pulsatile nature of the micropumps is to employ flowrate profiles. The peritoneal dialysis treatment of the present disclosure includes the cyclical introduction and removal of dialysis fluid to and from the patient's peritoneal cavity. Excess pressure in either direction may cause patient discomfort. System 10 and associated methodology however capitalize on the phenomenon that the patient is most susceptible to discomfort at the beginning and end of both patient fills and patient drains. During the middle of both the fills and drains, patient discomfort is less likely and/or less intense.

It is accordingly contemplated to program control unit 50 to store flowrate profiles that cause the micropump having fluid portion 110 or 150 to begin a patient fill or drain at a lower flowrate until a certain amount of fresh or used dialysis fluid is delivered to or removed from the patient, e.g., the first ten percent of a total fill or drain volume. After the initial defined amount of fresh or used dialysis fluid is delivered or removed, control unit 50 running the appropriate profile causes the micropump having fluid portion 110 or 150 to ramp the flowrate up to full speed according to a desired acceleration curve, e.g., linear or sinusoidal. At a certain point near the end of the fill or drain, e.g., the last ten percent, control unit 50 running the appropriate profile causes the micropump having fluid portion 110 or 150 to ramp the flowrate down to the same or different lower flowrate according to a desired deceleration curve, e.g., linear or sinusoidal. The system and methodology of the present disclosure may employ the flowrate profiles alone or in combination with pressure dampeners or pressure sensors or pods 102a and 102b to address the pulsatile input and output nature of the micropumps of the present disclosure.

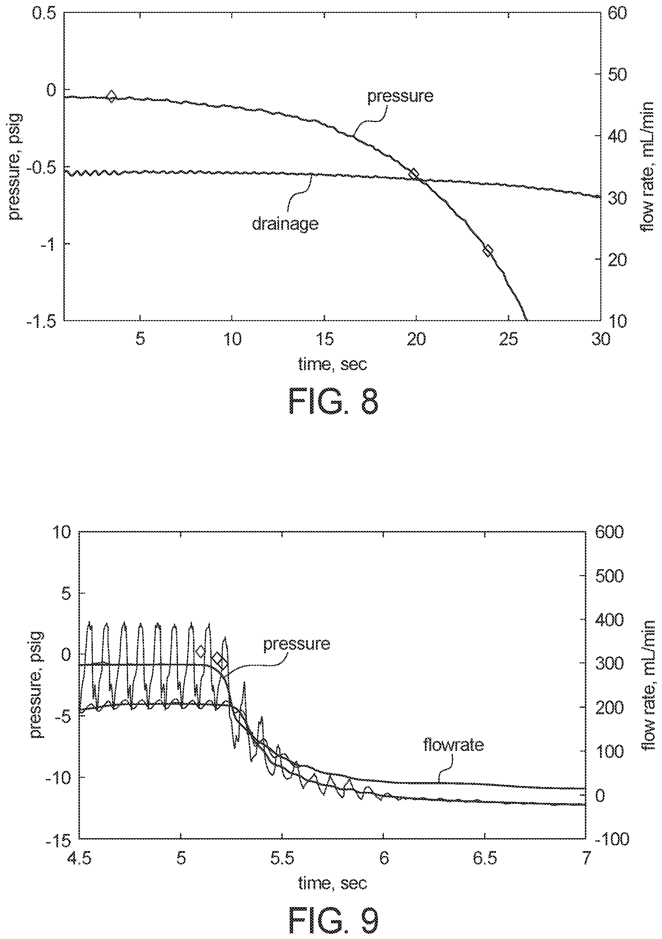

Referring now to Fig. 8, an example plot illustrates that at a drain flowrate (labeled "drainage") of about 34mL/min, the patient becomes close to empty as the micropump, e.g., using micropump head 110, tries to maintain a constant flowrate by increasing suction at its inlet owing to increased catheter resistance. The negative pressure increase (labeled "pressure") as measured by the pressure sensor or pods 102a or 102b located between micropump head 110 is detected by control unit 50, which is programmed to look for such negative pressure increase during the patient drain. For example, control unit 50 for any of the versions of system 10 discussed herein may be programmed to look for the negative pressure to (i) reach a certain limit, (ii) change at a certain rate or slope, or (iii) combinations thereof. Once the limit or criterion is reached, control unit 50 causes the patient drain to stop. Control unit 50 notes the volume of the drain for use in determining UF as discussed above.

It should be noted that the treatment algorithm stored on control unit 50 may employ checks for occlusion before a patient drain is considered complete or that the patient is considered to be empty enough to stop the current drain phase.

Referring now to Fig. 9, an example plot illustrates the detection of a negative pressure drain occlusion in patient line 154 during a patient drain using any version of system 10 discussed herein, e.g., using micropump head 110. Here, when patient line 154 becomes kinked, the flowrate (labeled "flowrate") drops and the negative suction pressure (labeled "pressure") increases (becomes more negative). The pressure change is measured by the pressure sensor 102a or 102b located between micropump head 110 and patient line 154 and is outputted to control unit 50, which is programmed to look for the flowrate drop and/or negative pressure increase. For example, control unit 50 for any of the versions of system 10 discussed herein may be programmed to look for a combination of (i) the flowrate dropping only (to a threshold level and/or over a certain rate of change), (ii) the negative pressure increasing only (to a threshold level and/or over a certain rate of change) in combination with the negative pressure increase occurring at a time unlikely to be and end of drain, or (iii) the flowrate dropping (to a threshold level and/or over a certain rate of change) in combination with the negative pressure increasing (to a threshold level and/or over a certain rate of change). Once any of (i) to (iii) above is triggered, control unit 50 determines an occlusion and sounds an alarm, perhaps after pushing fluid back towards the patient to confirm the occlusion. Control unit 50 also notes the volume of the drain up until the occlusion. Control unit may also cause user interface 60 to display an audio, visual or audiovisual message showing the patient how to clear the occlusion so that the patient drain may resume.

It should be noted that since the devices illustrated herein do not directly measure flowrate other than expected stroke volume discussed herein, pressure decay is the indicator for negative pressure drain occlusion. It is contemplated in an alternative to add one or more flow sensor outputting to control unit 50 in any of the implementations discussed hereon. Here, the flowrate sensor output may be used alternatively to or additionally with the pressure decay the indicator for negative pressure drain occlusion just described.

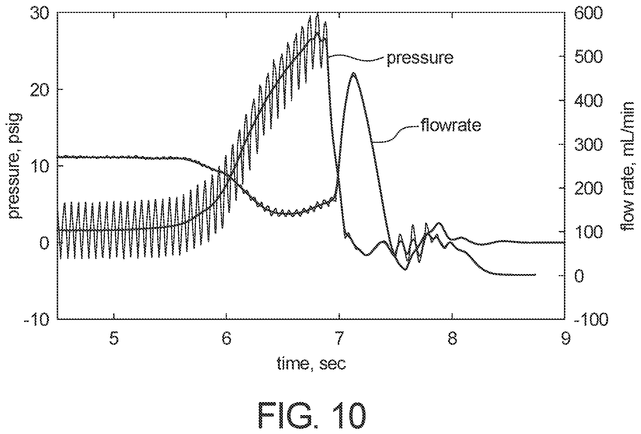

Referring now to Fig. 10, an example plot illustrates the detection of a positive pressure occlusion in patient line 154 during a patient fill using any version of system 10 discussed herein, e.g., using micropump head 110. Here, when patient line 154 becomes kinked, the flowrate (labeled "flowrate") drops and the positive fill pressure (labeled "pressure") spikes (becomes more positive). The pressure change is again measured by pressure sensor 102a or 102b located between fluid micropump potion 110 and patient line 154 and is outputted to control unit 50, which is programmed to look for the flowrate drop and/or positive pressure increase. For example, control unit 50 for any of the versions of system 10 discussed herein may be programmed to look for a combination of (i) the flowrate dropping only (to a threshold level and/or over a certain rate of change), (ii) the positive pressure increasing only (to a threshold level and/or over a certain rate of change), or (iii) the flowrate dropping (to a threshold level and/or over a certain rate of change) in combination with the positive pressure increasing (to a threshold level and/or over a certain rate of change). Once any of (i) to (iii) above is triggered, control unit 50 determines an occlusion and sounds an alarm, perhaps after pushing fluid back towards the patient to confirm the occlusion. Control unit 50 also notes the volume of the fill up until the occlusion. Control unit may also cause user interface 60 to display an audio, visual or audiovisual message showing the patient how to clear the occlusion so that the patient fill may resume.

As illustrated above, micropump system 10 provides certain advantages regarding empty and occlusion detection. Empty and occlusion detection based on pressure decay or rise as described herein provides quick results. Partial occlusions, which may result in slower flowrates in other types of APD systems, do not affect micropump system 10 similarly, because the micropump system is able to maintain required flowrates even with the partial occlusion due to its higher pressure vs accuracy capability, thereby maintaining a prescribed treatment time or improving treatment time compared to other types of systems.

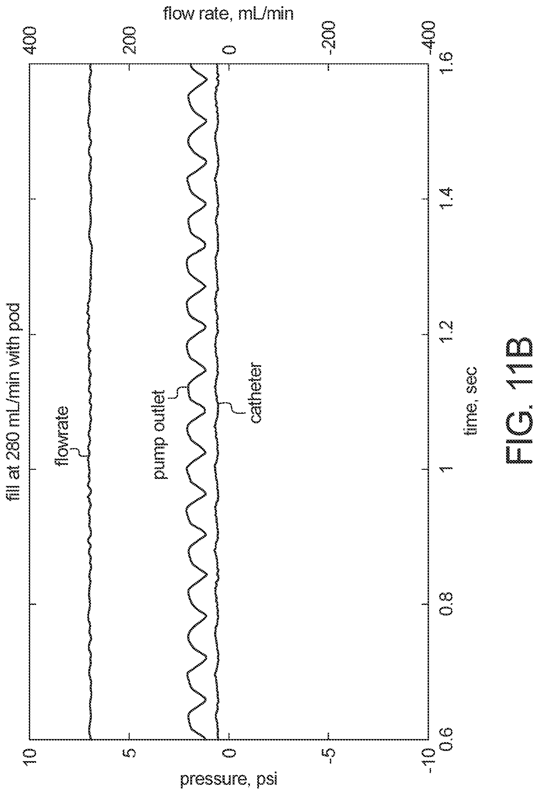

Regarding the dampening effects of pressure pods 102a and 102b discussed herein, Figs. 11A and 11B illustrate the beneficial dampening effects for a larger patient fill flowrate of 280 mL/min. The curves of Fig. 11A are for actuation of the micropump without a pressure pod 102a or 102b, while the curves of Fig. 11B are for actuation of the micropump with a pressure pod 102a or 102b. The plots of Figs. 11A and 11B each show three curves, namely, a "flowrate" curve, a "pump outlet" curve that shows pressure measurements taken at 0.3 meters from an outlet of micropump head 110, and a "catheter" curve that shows pressure measurements taken at 0.5 meters from a patient's indwelling catheter. Fig. 11B illustrates that the fill flowrate at 280 mL/min is relatively constant and is a great deal more constant than the fill flowrate at 280 mL/min in Fig. 11A. The amplitudes of the pump outlet pressure curve of Fig. 11B at 280 mL/min are quarter or less than the amplitudes of the pump outlet pressure curve of Fig. 11A at 280 mL/min. Likewise, the amplitudes of the catheter pressure curve of Fig. 11B at 280 mL/min are virtually flat relative to the amplitudes of the catheter pressure curve of Fig. 11A at 280 mL/min. Figs. 11A and 11B illustrate that pressure pods 102a or 102b have a significant impact on dampening the pulsatile output of the micropumps of the present disclosure while filling the patient at 280 mL/min.

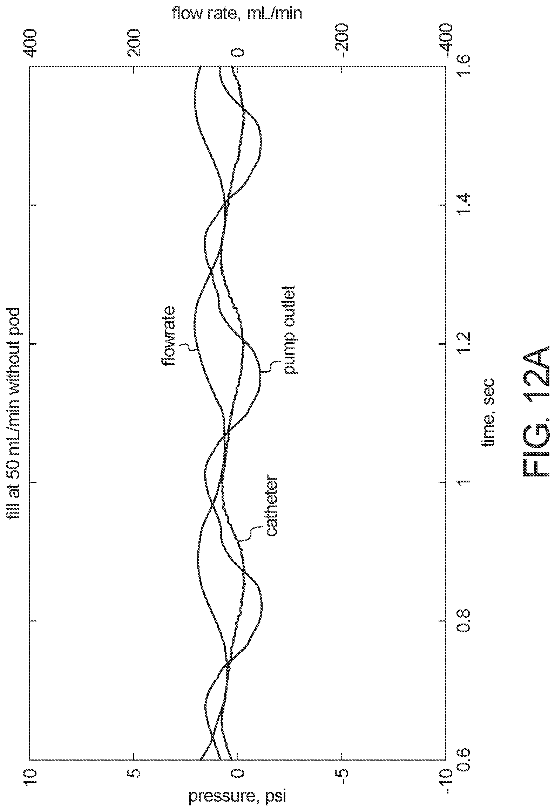

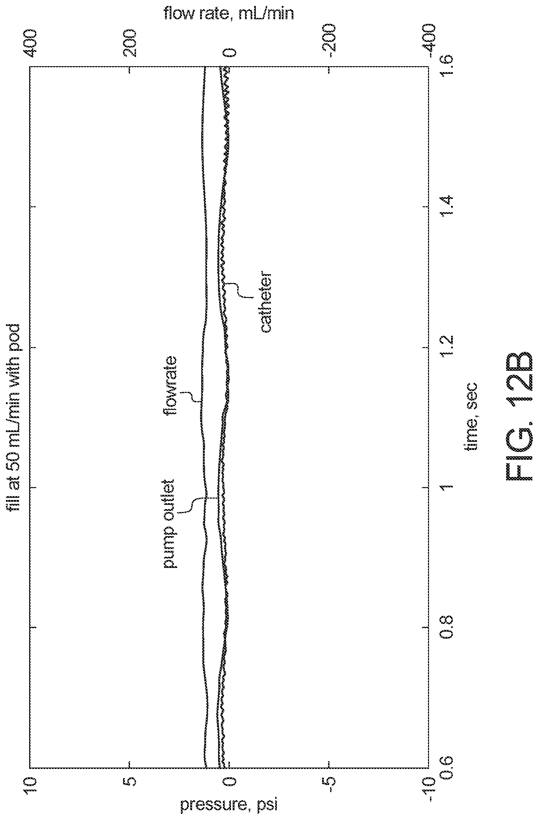

Figs. 12A and 12B illustrate the beneficial dampening effects for a low patient fill flowrate of 50 mL/min (e.g., at the end of a patient fill or for a pediatric patient fill). The curves of Fig. 12A are for actuation of the micropump without a pressure pod 102a or 102b, while the curves of Fig. 12B are for actuation of the micropump with a pressure pod 102a or 102b. The plots of Figs. 12A and 12B show the same three "flowrate" curve, "pump outlet" curve and "catheter" curve described above for Figs. 11A and 11B. Fig. 12B illustrates that the fill flowrate at 50 mL/min is relatively constant and a great deal more constant than the fill flowrate at 50 mL/min in Fig. 12A. The amplitudes of the pump outlet pressure curve of Fig. 12B at 50 mL/min are virtually flat relative to the amplitudes of the pump outlet pressure curve of Fig. 12A at 50 mL/min. Likewise, the amplitudes of the catheter pressure curve of Fig. 12B at 50 mL/min are virtually flat relative to the amplitudes of the catheter pressure curve of Fig. 12A at 50 mL/min. Figs. 12A and 12B illustrate that pressure pods 102a or 102b have a significant impact on dampening the pulsatile output of the micropumps of the present disclosure while filling the patient at 50 mL/min.

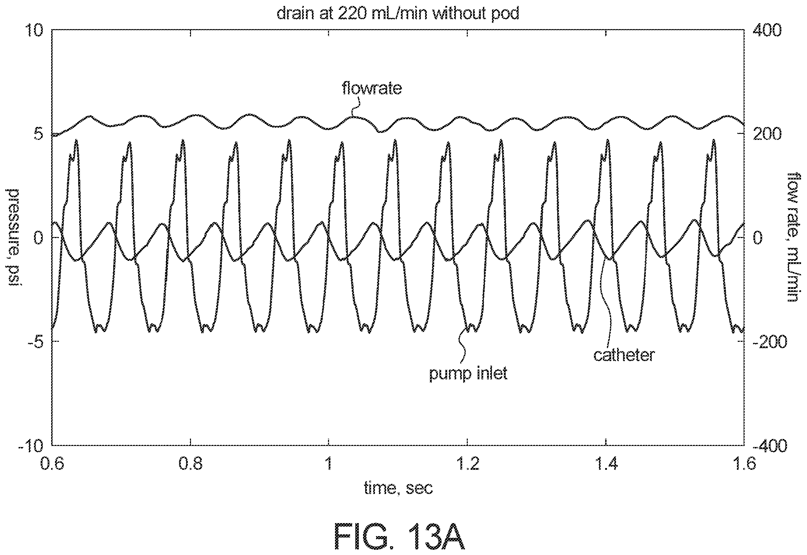

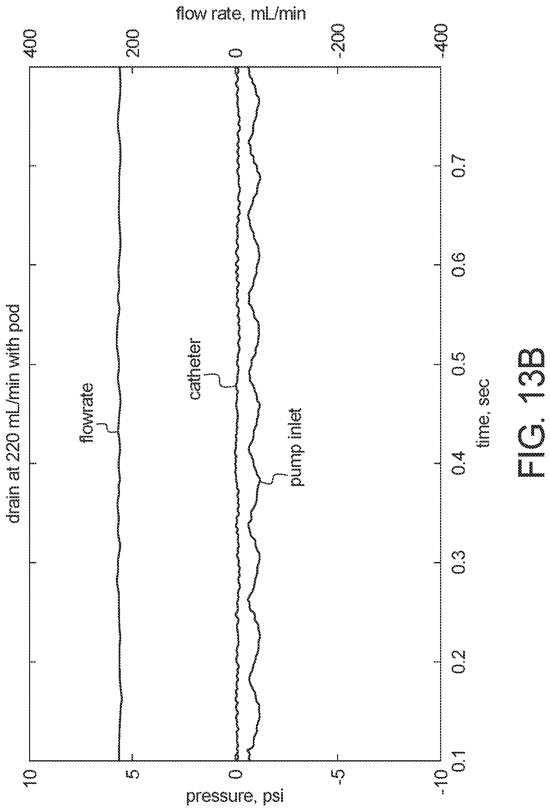

Figs. 13A and 13B illustrate the beneficial dampening effects for a larger patient drain flowrate of 220 mL/min. The curves of Fig. 13A are for actuation of the micropump without a pressure pod 102a or 102b, while the curves of Fig. 13B are for actuation of the micropump with a pressure pod 102a or 102b. The plots of Figs. 13A and 13B show three "flowrate" curve, "pump inlet" curve and "catheter" curve described above for Figs. 11A and 11B. Fig. 13B illustrates that the drain flowrate at 220 mL/min is relatively constant and a great deal more constant than the drain flowrate at 220 mL/min in Fig. 13A. The amplitudes of the pump inlet pressure curve of Fig. 13B at 220 mL/min are a quarter or less than the amplitudes of the pump inlet pressure curve of Fig. 13A at 220 mL/min. Likewise, the amplitudes of the catheter pressure curve of Fig. 13B at 220 mL/min are virtually flat relative to the amplitudes of the catheter pressure curve of Fig. 13A at 220 mL/min. Figs. 13A and 13B illustrate that pressure pods 102a or 102b have a significant impact on dampening the pulsatile output of the micropump while draining the patient at 220 mL/min.

It should be understood that various changes and modifications to the devices described herein will be apparent to those skilled in the art. For example, instead of providing lids 26a and 26b that open to allow disposable set 100a to be translated down into APD cycler 20, disposable set 100a could instead be loaded directly onto the top of the cycler. Also, while stopcock and pinch valves have been illustrated, it is contemplated to alternatively provide cassette-based valves, such as pneumatic valves. Moreover, any of the three-way valves described herein may be replaced with multiple two-way valves.

## Claims

1. A peritoneal dialysis ("PD") system (10) comprising:
a cycler (20) including
a micropump actuator (30),
an inline fluid heater or batch fluid heater (46),
a pressure transducer (36), and
at least one valve actuator (40);
a disposable set (100a) including
a micropump head (110) sized and shaped for mating with and being cyclically driven by the micropump actuator (30),
a pressure sensor (102) configured to operably communicate with the pressure transducer (36), the pressure sensor positioned and arranged to dampen pressure fluctuations of fresh or used dialysis fluid flowing between the micropump head and a patient line,
at least one fluid valve portion (130) or a portion of at least one fluid line for interfacing with the at least one valve actuator (40), and
an inline fluid heating pathway (140) or a batch heater container; and
a control unit (50), wherein the disposable set is arranged to allow, and the control unit is programmed to operate the micropump actuator and the at least one valve actuator so that fresh and used dialysis fluid flows through the micropump head in a same direction,
wherein the inline fluid heater or the batch fluid heater of the cycler is positioned and arranged to heat the inline fluid heating pathway or the batch heater container of the disposable set, respectively, when the disposable set is mounted to the cycler.

2. The PD system of Claim 1, wherein the micropump actuator and the micropump head are more accurate in the same direction than in a reverse direction.

3. The PD system of Claim 1, wherein the micropump actuator includes a keyed driver and the micropump head includes a keyed recess sized and shaped for mating with the keyed driver of the micropump actuator.

4. The PD system of Claim 1, wherein the pressure sensor includes a fluid pressure chamber (104) having a sealed diaphragm configured to fluctuate due to pressure variations, and wherein the cycler includes a transmission fluid chamber (106) positioned and arranged to mate with the fluid pressure chamber when the disposable set is mounted to the cycler.

5. The PD system of Claim 4, wherein a transmission fluid line extends from the transmission fluid chamber to the pressure transducer.

6. The PD system of Claim 1, wherein the pressure sensor includes a fluid pressure chamber (104) having a sealed diaphragm configured to fluctuate due to pressure variations, and a transmission fluid chamber (106) sealed to the fluid pressure chamber.

7. The PD system of Claim 6, wherein the pressure sensor interfaces with the pressure transducer via a transmission fluid line placed in fluid communication with the transmission fluid chamber.

8. The PD system of Claim 1, wherein the at least one fluid valve portion includes a rotating stem and the at least one valve actuator is configured to rotate the rotating stem.

9. The PD system of Claim 1, wherein the at least one valve actuator is a pinch valve positioned and arranged to occlude the portion of at least one fluid line when the disposable set is mounted to the cycler.

10. The PD system of Claim 1, wherein the disposable set being arranged to allow fresh and used dialysis fluid to flow through the micropump head in a same direction includes disposing both a patient line and a drain line of the disposable set, at least during a patient fill phase, downstream from the micropump head.

11. The PD system of Claim 1, wherein the control unit is programmed to operate the micropump actuator so that at least one of fresh or used dialysis fluid flows at a lower flowrate at a beginning of at least one of a patient fill or a patient drain, respectively, than during a middle portion of the at least one of the patient fill or the patient drain.

12. The PD system of Claim 11, wherein the control unit is further programmed to operate the micropump actuator so that the at least one of fresh or used dialysis fluid flows at a lower flowrate at an end of the at least one of the patient fill or the patient drain, respectively, than during a middle portion of the at least one of the patient fill or the patient drain.

13. The PD system of Claim 1, wherein the control unit is programmed to monitor a negative pressure from the pressure transducer during a patient drain in which the micropump actuator actuates the micropump head to remove used dialysis fluid from a patient, and wherein when the sensed negative pressure increases to a predefined extent, the control unit ends the patient drain.

14. The PD system of Claim 13, wherein the predefined extent includes (i) a threshold value, (ii) a threshold rate of change of negative pressure, or (iii) a combination of (i) and (ii).

## Patentansprüche

1. Peritonealdialysesystem ("PD"-System) (10), umfassend:
einen Cycler (20), einschließend
ein Mikropumpenbetätigungselement (30),
einen Inline-Fluiderhitzer oder Chargenfluiderhitzer (46),
einen Druckwandler (36), und
mindestens ein Ventilbetätigungselement (40);
einen Einwegsatz (100a), einschließend
einen Mikropumpenkopf (110), der bemessen und geformt ist zum Zusammenpassen mit dem Mikropumpenbetätigungselement (30) und um durch diesen zyklisch angetrieben zu werden,
einen Drucksensor (102), der konfiguriert ist, um mit dem Druckwandler (36) betriebsfähig zu kommunizieren, wobei der Drucksensor positioniert und angeordnet ist, um Druckschwankungen von frischem oder gebrauchtem Dialysefluid, das zwischen dem Mikropumpenkopf und einer Patientenleitung fließt, abzudämpfen,
mindestens einen Fluidventilteil (130) oder einen Teil mindestens einer Fluidleitung zum Verbinden mit dem mindestens einen Ventilbetätigungselement (40), und
einen Inline-Fluidheizpfad (140) oder einen Chargenerhitzerbehälter; und
eine Steuereinheit (50), wobei der Einwegsatz angeordnet ist, um zu ermöglichen, und die Steuereinheit programmiert ist, um das Mikropumpenbetätigungselement und das mindestens eine Ventilbetätigungselement zu betreiben, sodass frisches und gebrauchtes Dialysefluid in einer gleichen Richtung durch den Mikropumpenkopf hindurch fließen,
wobei der Inline-Fluiderhitzer oder der Chargenfluiderhitzer des Cyclers positioniert und angeordnet ist, um den Inline-Fluidheizpfad beziehungsweise den Chargenerhitzerbehälter des Einwegsatzes zu erhitzen, wenn der Einwegsatz an dem Cycler montiert ist.

2. PD-System nach Anspruch 1, wobei das Mikropumpenbetätigungselement und der Mikropumpenkopf in der gleichen Richtung genauer sind als in einer Rückwärtsrichtung.

3. PD-System nach Anspruch 1, wobei das Mikropumpenbetätigungselement einen verkeilten Mitnehmer einschließt und der Mikropumpenkopf eine verkeilte Aussparung einschließt, die zum Zusammenpassen mit dem verkeilten Mitnehmer des Mikropumpenbetätigungselements bemessen und geformt ist.

4. PD-System nach Anspruch 1, wobei der Drucksensor eine Fluiddruckkammer (104) einschließt, die eine abgedichtete Membran aufweist, die konfiguriert ist, um aufgrund von Druckvariationen zu schwanken, und wobei der Cycler eine Übertragungsfluidkammer (106) einschließt, die positioniert und angeordnet ist, um mit der Fluiddruckkammer zusammenzupassen, wenn der Einwegsatzes an dem Cycler montiert ist.

5. PD-System nach Anspruch 4, wobei sich eine Übertragungsfluidleitung von der Übertragungsfluidkammer zu dem Druckwandler erstreckt.

6. PD-System nach Anspruch 1, wobei der Drucksensor eine Fluiddruckkammer (104), die eine abgedichtete Membran aufweist, die konfiguriert ist, um aufgrund von Druckvariationen zu schwanken, und eine Übertragungsfluidkammer (106) einschließt, die gegen die Fluiddruckkammer abgedichtet ist.

7. PD-System nach Anspruch 6, wobei der Drucksensor über eine Übertragungsfluidleitung mit dem Druckwandler verbunden ist, die in Fluidkommunikation mit der Übertragungsfluidkammer platziert ist.

8. PD-System nach Anspruch 1, wobei der mindestens eine Fluidventilteil einen rotierenden Schaft einschließt und das mindestens eine Ventilbetätigungselement konfiguriert ist, um den rotierenden Schaft zu rotieren.

9. PD-System nach Anspruch 1, wobei das mindestens eine Ventilbetätigungselement ein Quetschventil ist, das positioniert und angeordnet ist, um den Teil der mindestens einen Fluidleitung zu verschließen, wenn der Einwegsatz an dem Cycler montiert ist.

10. PD-System nach Anspruch 1, wobei der Einwegsatz, der angeordnet ist, um zu ermöglichen, dass frisches und gebrauchtes Dialysefluid in einer gleichen Richtung durch den Mikropumpenkopf hindurch fließen, ein Einrichten sowohl einer Patientenleitung als auch einer Entleerungsleitung des Einwegsatzes, mindestens während einer Patientenbefüllungsphase, stromabwärts von dem Mikropumpenkopf einschließt.

11. PD-System nach Anspruch 1, wobei die Steuereinheit programmiert ist, um das Mikropumpenbetätigungselement zu betreiben, sodass mindestens eines von frischem oder gebrauchtem Dialysefluid zu einem Beginn von mindestens einem von einer Patientenbefüllung beziehungsweise einer Patientenentleerung mit einer niedrigeren Flussrate als während eines mittleren Teils des mindestens einen von der Patientenbefüllung oder der Patientenentleerung fließt.

12. PD-System nach Anspruch 11, wobei die Steuereinheit ferner programmiert ist, um das Mikropumpenbetätigungselement zu betreiben, sodass das mindestens eine von frischem oder gebrauchtem Dialysefluid an einem Ende der mindestens einen von der Patientenbefüllung oder der Patientenentleerung jeweils mit einer niedrigeren Flussrate als während eines mittleren Teils der mindestens einen von der Patientenbefüllung oder der Patientenentleerung fließt.

13. PD-System nach Anspruch 1, wobei die Steuereinheit programmiert ist, um einen Unterdruck von dem Druckwandler während einer Patientenentleerung zu überwachen, bei der das Mikropumpenbetätigungselement den Mikropumpenkopf betätigt, um gebrauchtes Dialysefluid aus einem Patienten zu entfernen, und wobei, wenn der erfasste Unterdruck auf ein vordefiniertes Ausmaß ansteigt, die Steuereinheit die Patientenentleerung beendet.

14. PD-System nach Anspruch 13, wobei das vordefinierte Ausmaß (i) einen Schwellenwert, (ii) eine Schwellenrate einer Änderung des Unterdrucks oder (iii) eine Kombination aus (i) und (ii) einschließt.

## Revendications

1. Système de dialyse péritonéale (« DP ») (10) comprenant :
un cycleur (20) comportant
un actionneur de micropompe (30),
un réchauffeur de fluide en ligne ou un réchauffeur de fluide discontinu (46),
un transducteur de pression (36), et
au moins un actionneur de valve (40) ;
un ensemble jetable (100a) comportant
une tête de micropompe (110) dimensionnée et conformée pour s'accoupler avec et être entraînée cycliquement par l'actionneur de micropompe (30),
un capteur de pression (102) configuré pour communiquer de manière fonctionnelle avec le transducteur de pression (36), le capteur de pression étant positionné et agencé pour amortir les fluctuations de pression du fluide de dialyse frais ou usagé s'écoulant entre la tête de micropompe et une ligne de patient,
au moins une partie de vanne de fluide (130) ou une partie d'au moins une conduite de fluide pour s'interfacer avec l'au moins un actionneur de vanne (40), et
un trajet de chauffage de fluide en ligne (140) ou un récipient de chauffage discontinu ; et
une unité de commande (50), dans lequel l'ensemble jetable est agencé pour permettre, et l'unité de commande est programmée pour actionner l'actionneur de micropompe et l'au moins un actionneur de vanne de sorte que le fluide de dialyse frais et usagé s'écoule à travers la tête de micropompe dans une même direction,
dans lequel le dispositif de chauffage de fluide en ligne ou le dispositif de chauffage de fluide par lots du cycleur est positionné et agencé pour chauffer le trajet de chauffage de fluide en ligne ou le récipient de chauffage par lots de l'ensemble jetable, respectivement, lorsque l'ensemble jetable est monté sur le cycleur.

2. Système PD selon la revendication 1, dans lequel l'actionneur de micropompe et la tête de micropompe sont plus précis dans le même sens que dans un sens inverse.

3. Système PD de la revendication 1, dans lequel l'actionneur de micropompe comprend un organe d'entraînement claveté et la tête de micropompe comprend un évidement claveté dimensionné et profilé pour s'accoupler avec l'organe d'entraînement claveté de l'actionneur de micropompe.

4. Système PD selon la revendication 1, dans lequel le capteur de pression comprend une chambre de pression de fluide (104) ayant un diaphragme scellé configuré pour fluctuer en raison des variations de pression, et dans lequel le cycleur comprend une chambre à fluide de transmission (106) positionnée et agencée pour s'accoupler avec la chambre de pression de fluide lorsque l'ensemble jetable est monté sur le cycleur.

5. Système de DP selon la revendication 4, dans lequel une conduite de fluide de transmission s'étend depuis la chambre à fluide de transmission jusqu'au transducteur de pression.

6. Système PD selon la revendication 1, dans lequel le capteur de pression comprend une chambre de pression de fluide (104) comportant une membrane étanche configurée pour fluctuer en raison des variations de pression, et une chambre à fluide de transmission (106) scellée à la chambre de pression de fluide.

7. Système PD de la revendication 6, dans lequel le capteur de pression est en interface avec le transducteur de pression par l'intermédiaire d'une ligne de fluide de transmission placée en communication fluidique avec la chambre à fluide de transmission.

8. Système PD selon la revendication 1, dans lequel l'au moins une partie de vanne de fluide comprend une tige rotative et l'au moins un actionneur de vanne est configuré pour faire tourner la tige rotative.

9. Système PD selon la revendication 1, dans lequel l'au moins un actionneur de vanne est une vanne à pincement positionnée et agencée pour occlure la partie d'au moins une ligne de fluide lorsque l'ensemble jetable est monté sur le cycleur.

10. Système de DP de la revendication 1, dans lequel l'ensemble jetable étant agencé pour permettre au fluide de dialyse frais et usagé de s'écouler à travers la tête de micropompe dans une même direction comprend la disposition à la fois d'une ligne de patient et d'une ligne de vidange de l'ensemble jetable, au moins pendant une phase de remplissage du patient, en aval de la tête de micropompe.

11. Système de DP selon la revendication 1, dans lequel l'unité de commande est programmée pour actionner l'actionneur de micropompe de sorte qu'au moins l'un d'un fluide de dialyse frais ou usagé s'écoule à un débit inférieur au début d'au moins l'un d'un remplissage du patient ou d'un drainage du patient, respectivement, que pendant une partie médiane de l'au moins un du remplissage du patient ou du drainage du patient.

12. Système de DP selon la revendication 11, dans lequel l'unité de commande est en outre programmée pour faire fonctionner l'actionneur de micropompe de sorte que l'au moins un parmi le fluide de dialyse frais ou usagé s'écoule à un débit inférieur à une fin de l'au moins un parmi le remplissage du patient ou le drainage du patient, respectivement, que pendant une partie médiane de l'au moins un parmi le remplissage du patient ou le drainage du patient.

13. Système de DP selon la revendication 1, dans lequel l'unité de commande est programmée pour surveiller une pression négative provenant du transducteur de pression pendant un drainage du patient dans lequel l'actionneur de micropompe actionne la tête de micropompe pour retirer le fluide de dialyse usagé d'un patient, et dans lequel, lorsque la pression négative détectée augmente dans une mesure prédéfinie, l'unité de commande met fin au drainage du patient.

14. Système PD selon la revendication 13, dans lequel l'étendue prédéfinie comprend (i) une valeur seuil, (ii) un taux de variation seuil de pression négative, ou (iii) une combinaison de (i) et de (ii).
